(19) Europäisches Patentamt
European Patent Office
Office européen des brevets

(11) Veröffentlichungsnummer: **0 246 580 B1**

# (12) EUROPÄISCHE PATENTSCHRIFT

(45) Veröffentlichungstag der Patentschrift: **03.07.91**

(51) Int. Cl.5: **C07D 233/58**, C08F 26/06

(21) Anmeldenummer: **87107116.3**

(22) Anmeldetag: **16.05.87**

## (54) Verfahren zur Herstellung von 3-Methyl-1-vinyl-imidazoliumchloriden und ihre Verwendung zur Herstellung von Polymerisaten.

(30) Priorität: **21.05.86 DE 3617069**

(43) Veröffentlichungstag der Anmeldung:
**25.11.87 Patentblatt 87/48**

(45) Bekanntmachung des Hinweises auf die Patenterteilung:
**03.07.91 Patentblatt 91/27**

(84) Benannte Vertragsstaaten:
**AT BE CH DE ES FR GB GR IT LI NL SE**

(56) Entgegenhaltungen:
**DE-A- 2 829 137**
**GB-A- 626 475**

**POLYMER, Band 14, Dezember 1973, Seiten 639-644; J.C. SALAMONE et al.: "Synthesis and homopolymerization studes of vinylimidazolium salts"**

**JOURNAL OF POLYMER SCIENCES: SYMPOSIUM Nr. 45, 1974, Seiten 39-50; C.G. OVERBERGER et al.: "The effect of copolymers of u(5)-vinylimidazole and quaternary imidazolium salts on the hydrolysis rates of charged and neutral esters. I."**

(73) Patentinhaber: **BASF Aktiengesellschaft**
**Carl-Bosch-Strasse 38**
**W-6700 Ludwigshafen(DE)**

(72) Erfinder: **Goertz, Hans-Helmut. Dr.**
**Am Wurmberg 11**
**W-6713 Freinsheim(DE)**
Erfinder: **Straub, Ferdinand, Dr.**
**Ziegelstrasse 20**
**W-6832 Hockenheim(DE)**
Erfinder: **Sanner, Axel, Dr.**
**Lorscher Ring 2c**
**W-6710 Frankental(DE)**
Erfinder: **Kolb, Albrecht, Dr.**
**Im Mandelgarten 9**
**W-6719 Weisenheim(DE)**
Erfinder: **Raubenheimer, Hans-Juergen**
**Benzstrasse 6**
**W-6834 Ketsch(DE)**
Erfinder: **Vogel, Friedrich, Dr.**
**Im kleinen Letten 3**
**W-6706 Wachenheim(DE)**

**EP 0 246 580 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Herstellung von 3-Methyl-1-vinyl-imidazoliumchloriden durch Quaternisierung von N-Vinylimidazolen in wäßriger Lösung mit Methylchlorid und ihre Verwendung zur Herstellung von wasserlöslichen Homo- oder Copolymerisaten.

Die Herstellung von wasserlöslichen kationischen Polymeren mit 3-Methyl-1-vinyl-imidazoliumchloriden als monomeres Strukturelement kann prinzipiell auf zwei Wegen erfolgen:
Man homo- oder copolymerisiert das entsprechende N-Vinylimidazol und quaternisiert anschließend das erhaltene Polymerisat mit einem Methylierungsreagens. Dieser Weg wird beispielsweise in J. Phys. Chem. 61, 1347-1352 (1957) beschrieben, wobei in Methanol gelöstes Polyvinylimidazol mit Methyliodid umgesetzt wird.

Man kann die Quaternisierung auch auf der Stufe des Monomeren durchführen und das erhaltene quaternäre Monomere bei der Polymerisation einsetzen. Auch hierfür sind aus der Literatur Beispiele bekannt. So beschreiben beispielsweise J. C. Salamone und Mitarbeiter in einer Reihe von Arbeiten, ACS Polymer Preprints 15, 462-467 (1974) und 13, 271-272 (1972), die Umsetzung von N-Vinylimidazolen mit Methyliodid oder Dimethylsulfat zu quartären Ammoniumsalzen. Sie gehen dabei so vor, daß sie die Monomeren, beispielsweise N-Vinylimidazol, in Ethylacetat als Lösungsmittel mit dem Alkylierungsreagens umsetzen und die entstehenden Salze als Feststoffe isolieren. Für die Polymerisation werden die Salze zusammen mit geeigneten Monomeren wieder aufgelöst.

Die Quaternisierung von N-Vinylimidazol mit Dimethylsulfat oder Methyliodid ist gängig und wird in der Literatur häufig beschrieben. Die Verwendung von Methylchlorid als Methylierungs- bzw. Quaternisierungsreagens dagegen ist ungewöhnlich, obwohl die erhaltenen Methochloride den Vorteil eines geringeren Molgewichts und damit einer höheren Ladungsdichte aufweisen. In Khim. Geterotsiklich. Soed. 7, Seiten 958-960 (1971) wird die Quaternisierung von Vinylimidazol mit Alkyliodiden, beispielsweise Methyliodid, und Alkylbromiden ohne Zugabe von Lösungsmittel beschrieben und dabei festgestellt, daß Vinylimidazol nicht mit Alkylchloriden reagiert, obwohl aus der GB-PS 626 475 bekannt ist, daß man z.B. 1-n-Decyl-2-methylimidazol im geschlossenen Gefäß mit Methylchlorid umsetzen kann.

Es ist allgemein bekannt, daß sich als Lösungsmittel für Quaternisierungen mit Alkylhalogeniden insbesondere polare Lösungsmittel, wie Nitromethan, Acetonitril oder Alkohole, eignen. Wasser erscheint dagegen im Hinblick auf die Unlöslichkeit oder geringe Löslichkeit der Alkylhalogenide in Wasser als weniger geeignet zu sein, wie beispielsweise aus Houben-Weyl, Methoden der organischen Chemie, Band XI/2, Seite 594-596 (1958) hervorgeht. Weiterhin wird dort ausgeführt, daß wegen der bedeutend geringeren Aktivität der Alkylchloride es zweckmäßig ist, die Alkylierung mit einem Alkylbromid oder Alkyliodid auszuführen und anschließend einen Halogenaustausch vorzunehmen.

Aufgabe der vorliegenden Erfindung ist es, ein möglichst einfach durchzuführendes technisches Verfahren zur Herstellung von 3-Methyl-1-vinyl-imidazoliumchloriden durch Umsetzung mit Methylchlorid in wäßriger Lösung zur Verfügung zu stellen.

Gegenstand der Erfindung ist ein Verfahren zur Herstellung von 3-Methyl-1-vinyl-imidazoliumchloriden der Formel I

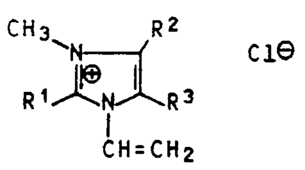

(I),

in der $R^1$, $R^2$ und $R^3$ H oder Methyl bedeuten, das dadurch gekennzeichnet ist, daß man eine 10- bis 70-gew.%ige, bevorzugt 25- bis 60-gew.%ige, wäßrige Lösung eines N-Vinylimidazols der Formel II

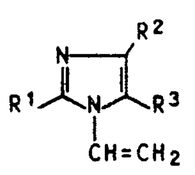

(II),

in der $R^1$, $R^2$ und $R^3$ H oder Methyl bedeuten, bei Temperaturen von 40 bis 100 °C, bevorzugt 50 bis 90 °C,

in einem geschlossenen System mit Methylchlorid in einer Menge von 90-110 Mol%, bevorzugt 94-100 Mol%, umsetzt.

Die bevorzugten Ausgangsverbindungen der Formel II sind 1-Vinylimidazol und 2-Methyl-1-vinylimidazol.

Die Umsetzung erfolgt in einer geschlossenen Apparatur, wobei zu der erhitzten Lösung des zu quaternisierenden Imidazols der Formel II das Methylchlorid so zudosiert wird, daß sich bei den angegebenen Temperaturen während der Reaktion Drucke von 15 bis 2 bar einstellen.

In Abhängigkeit insbesondere von der Reaktionsgeschwindigkeit, den Temperaturen und der verwendeten Apparatur wird innerhalb des genannten Druckbereiches mit einer entsprechenden Dosiergeschwindigkeit Methylchlorid zudosiert. In Apparaturen, die besonders für höhere Drucke geeignet und bei denen eine gute Wärmeabführung gewährleistet ist, kann innerhalb des beschriebenen Druckbereiches bei höheren Drucken und mit entsprechend stärkerer Zudosierung von Methylchlorid gearbeitet werden.

Wenn eine möglichst vollständige Quaternisierung des Imidazols erreicht werden soll, werden wenigstens die equimolare Menge Methylchlorid oder bis zu 10 Mol% Überschuß zugesetzt. Die bevorzugten Mengen Methylchlorid liegen bei 94 bis 100 Mol% und besonders bevorzugt bei 94 bis 99 Mol%. Da die Reaktionsgeschwindigkeit gegen Ende der Reaktion zusehends abnimmt, wird bei Verwendung eines geringen Unterschusses an Methylchlorid in der Regel eine vollständigere Umsetzung des Methylchlorids erreicht.

In einer weiteren Ausführungsform kann die eingesetzte wäßrige Lösung einen einwertigen aliphatischen Alkohol mit 1-3 C-Atomen als zusätzliches Lösungsmittel enthalten, und zwar vorteilhaft in einer Menge von 5-30 Gew.-%, bezogen auf das vorhandene Wasser. Hierfür kommen Methanol, Ethanol, n-Propanol und insbesondere Isopropanol in Betracht.

Der überraschende Effekt der erfindungsgemäßen Quaternisierungsreaktion liegt in der Einfachheit des Verfahrens. Es war nicht vorhersehbar, daß in Wasser als Lösungsmittel eine vollständige Umsetzung erreicht werden konnte, da aus dem Stand der Technik hervorgeht, daß eine direkte Umsetzung nicht oder nur sehr schwer möglich ist und andererseits Methylchlorid in Wasser nur eine sehr geringe Löslichkeit aufweist. Bei Raumtemperatur und 1 bar werden von 1 kg Wasser 9 g Methylchlorid, bei 60 °C nur noch 2,64 g gelöst, wie z.B. aus Ullmann, Enzyklopädie der Technischen Chemie, Bd. 9, S. 405 (1975), hervorgeht.

Die nach der erfindungsgemäßen Reaktion erhaltene wäßrige Lösung kann vorteilhafterweise direkt für die Herstellung entsprechend wasserlöslicher Polymerisate verwendet werden. In der Regel ist daher eine Isolierung der erhaltenen 3-Methyl-1-vinyl-imidazoliumchloride nicht erforderlich. Gegenstand der Erfindung ist daher auch die Verwendung der erhaltenen Lösungen nach Anspruch 1 zur Herstellung von wasserlöslichen Homo-oder Copolymerisaten von 1-Vinyl-imidazoliumchloriden der Formel I.

Zweckmäßigerweise werden die erhaltenen Lösungen der 3-Methyl-1-vinyl-imidazoliumchloride direkt oder mit anderen polymerisierbaren Monomeren unter Verwendung radikalbildender Initiatoren in an sich üblicher Weise in wäßrigen oder wäßrig-alkoholischen Lösungen polymerisiert.

Zweckmäßige Comonomere sind wasserlösliche ungesättigte Monomere, wie beispielsweise N-Vinylimidazole der oben genannten Formel II, Acrylamid und Methacrylamid, N-Methylolacrylamid und N-Methylolmethacrylamid, Hydroxyalkylacrylate und Hydroxyalkylmethacrylate mit 2 bis 4 C-Atomen im Hydroxyalkylrest, wie Hydroxyethyl(meth)acrylat, Hydroxypropyl(meth)acrylat, gegebenenfalls in Form ihrer technischen Gemische, Polyethylenglykol(meth)acrylate mit 2 bis 50 Ethylenoxideinheiten, und insbesondere N-Vinylamide, wie N-Vinylpyrrolidon, N-Vinylcaprolactam, N-Vinylformamid, N-Vinylacetamid oder N-Methyl-N-vinylacetamid.

Gegebenenfalls können auch wasserunlösliche Comonomere, wie Styrol, $\alpha$-Olefine, wie Penten oder Buten, Acryl- und Methacrylsäurealkylester mit 1 bis 18 C-Atomen im Alkyl. Carbonsäurevinylester mit 2 bis 10 C-Atomen im Carbonsäurerest, wie Vinylacetat oder Vinylpropionat, mit den erfindungsgemäß hergestellten 3-Methyl-1-vinyl-imidazoliumchloriden copolymerisiert werden. Dabei ist es vorteilhaft, wenn dem Quaternisierungsgemisch bereits eine solche Menge eines Alkohols zugesetzt worden ist, daß eine homogene Lösung entsteht.

Ganz besonders bevorzugt ist die Herstellung von Homopolymerisaten der 1-Vinyl-imidazoliumchloride der Formel I oder von wasserlöslichen Copolymeren aus 97 bis 10 Gew.% eines 3-Methyl-1-vinyl-imidazoliumchlorids der Formel I und 3 bis 90 Gew.% eines Monomeren aus der Gruppe N-Vinylpyrrolidon, 2-Hydroxyethylacrylat, 2-Hydroxyethylmethacrylat und Hydroxypropylacrylat (technisches Gemisch), bezogen auf das Gesamtgewicht der Monomeren.

Die Polymerisationen werden zweckmäßigerweise durchgeführt in 10 bis 50-gew.%igen wäßrigen oder wäßrigalkoholischen Lösungen, bevorzugt wäßrig-isopropanolischen Lösungen, in Gegenwart von radikalbildenden Initiatoren in einer Menge von 0,1 bis 2,0 bezogen auf das Gewicht der Monomeren, und bei

Temperaturen von 40 bis 100 °C.

Als radikalbildende Initiatoren können Wasserstoffperoxid oder anorganische Persulfate verwendet werden, ebenso organische Verbindungen vom Peroxid- oder Azotyp. Als organische Peroxide kommen beispielsweise Dicyclohexylperoxidicarbonat, Dibenzoylperoxid, Dilauroylperoxid, tert.-Butylperpivalat, tert.-Butyl-2-ethylhexanoat und als Azoverbindungen 2,2'-Azobisisobutyrodinitril, 2,2'-Azobis(2,4-dimethylvaleronitril), 2,2'-Azobis(2-amidinopropan)hydrochlorid oder 4,4'-Azobis(4-cyanopentansäure) in Betracht.

Eine Wasserlöslichkeit der verwendeten Radikalbildner ist nicht zwingend erforderlich. Wasserunlösliche Initiatoren können beispielsweise in einem niederen Alkohol gelöst oder ohne Lösungsmittel direkt zudosiert werden. Die Wahl des Initiators richtet sich zweckmäßigerweise u.a. nach den Polymerisationstemperaturen, die bevorzugt zwischen 40 und 100 °C liegen.

Das Molekulargewicht der Polymeren kann, falls wünschenswert, durch Zugabe von Reglern in die Reaktionsmischung erniedrigt werden. Als Regler kommen beispielsweise niedere Alkohole in Betracht. Wenn ihre Verwendung vorteilhaft ist, können die niederen Alkohole zweckmäßigerweise bereits bei der Herstellung der Imidazoliumchloride in der erforderlichen Menge zugefügt werden. Als Regler für das Molekulargewicht können aber auch andere üblicherweise zu diesem Zweck eingesetzte Verbindungen, wie Schwefelverbindungen, beispielsweise 2-Mercaptoethanol, Butylmercaptan, Dodecylmercaptan, Thioglykolsäure, Thioessigsäure, Thiomilchsäure, Halogenverbindungen, wie Tetrachlorkohlenstoff, 1,1,1-Tribrompropan, oder Ameisensäure und ihre Derivate eingesetzt werden. Durch die geeignete Wahl von Regler, Initiator, Polymerisationstemperatur und Monomerenkonzentration wird der K-Wert des erhaltenen Polymerisats, der ein Maß für das Molekulargewicht ist, eingestellt. Die K-Werte der erhaltenen Copolymerisate betragen zweckmäßigerweise 20 bis 150 und die der Homopolymerisate 15 bis 80, wobei die Messungen an einer 1-gew.%igen wäßrigen Lösung bei 25 °C vorgenommen werden.

Die erhaltenen Polymeren können vielfältig verwendet werden, beispielsweise als Leitfähigkeitsharze, als Flockungsmittel, als Haarkonditionierungsmittel, als Hilfsmittel bei der Ölgewinnung und anderes mehr.

Die folgenden Beispiele erläutern die Erfindung. Die Hydrierjodzahl wird gemäß DIN-Vorschrift 53 241 und der K-Wert jeweils an einer 1-gew.%igen Lösung in Wasser bei 25 °C gemäß Fikentscher gemessen.

Beispiel 1

In einem 6 l-Rührbehälter werden 2 kg 2-Methyl-1-vinylimidazol und 1,5 kg Wasser unter Rühren auf 75 °C aufgeheizt. Dann wird bei geschlossener Apparatur soviel Chlormethan zudosiert, daß der Druck auf ca. 4 bar ansteigt. Man wartet, bis der Druck auf ca. 2 bar abgefallen ist und dosiert dann wieder Chlormethan zu. Das wiederholt man solange, bis 450 g Chlormethan (96 Mol%, bezogen auf 2-Methyl-1-vinyl-imidazol) umgesetzt sind. Nach dem Abkühlen erhält man eine 58-gew.%ige Lösung des quaternierten Monomeren. Die Hydrierjodzahl der Lösung beträgt 90 (theoretisch 92).

Beispiel 2

In einem 6 l-Rührbehälter werden 1 kg 2-Methyl-1-vinylimidazol, 0,21 kg Isopropanol und 0,84 kg Wasser gegeben und unter Rühren auf 75 °C aufgeheizt. Dann wird wie in Beispiel 1 verfahren, bis 440 g Chlormethan (94 Mol%. bezogen auf 3-Methyl-1-vinylimidazol)umgesetzt sind. Die erhaltene Lösung hat einen Feststoffgehalt von 58 %. Die Hydrierjodzahl beträgt 88 (theoretisch 92).

Beispiel 3

In einem 6 l-Rührbehälter werden 1 kg 1-Vinyl-imidazol und 1,05 kg Wasser gegeben und unter Rühren auf 75 °C aufgeheizt. Dann wird wie in Beispiel 1 verfahren, bis 525 g Chlormethan (98 Mol%, bezogen auf 1-Vinylimidazol) umgesetzt sind. Die erhaltene Lösung hat einen Feststoffgehalt von 59 %. Die Hydrierjodzahl der Lösung beträgt 104 (theoretisch 103).

Beispiel 4

In einem 6 l-Rührbehälter wird eine Lösung von 1 kg 1-Vinylimidazol, 0,21 kg Isopropanol und 0,84 kg Wasser unter Rühren auf 73 °C aufgeheizt. Dann wird wie in Beispiel 1 verfahren, bis 520 g Chlormethan (97 Mol%, bezogen auf Vinylimidazol) umgesetzt sind. Die erhaltene Lösung hat einen Feststoffgehalt von 59 %. Die Hydrierjodzahl beträgt 104 (theoretisch 103).

Polymerisationsbeispiele

Beispiel 5

Eine Mischung aus 203 g Monomerlösung gemäß Beispiel 1, 100 g Wasser und 280 g Vinylpyrrolidon, im folgenden als Zulauf 1 bezeichnet, wird mit konzentrierter Ammoniaklösung auf einen pH-Wert von 7,5 eingestellt. Aus 2 g 2,2'-Azobis(2-amidinopropan)hydrochlorid und 55 g Wasser wird eine zweite Lösung, im folgenden als Zulauf 2 bezeichnet, bereitet.

In einem 2 l-Glasgefäß, das mit Rührer, Heizung, Rückflußkühler und Dosiervorrichtungen ausgestattet ist, werden 362 g Wasser, 50 ml Zulauf 1 und 5 ml Zulauf 2 unter Rühren auf 75° C erhitzt. Nach dem Erreichen der vorgesehenen Temperatur werden der restliche Zulauf 1 innerhalb von 4 Stunden und der restliche Zulauf 2 innerhalb von 5 Stunden bei gleichbleibender Temperatur von 75° C zudosiert. Anschließend wird noch eine Stunde lang bei dieser Temperatur gerührt. Man erhält eine klare, hochviskose Polymerlösung. Der K-Wert des Polymeren beträgt 141,5.

Beispiel 6

Eine Mischung aus 483 g Monomerlösung gemäß Beispiel 3, 15 g Vinylpyrrolidon und 137 g Wasser wird mit konzentrierter Ammoniaklösung auf einen pH-Wert von 7,5 eingestellt (Zulauf 1). Aus 3 g 2,2'-Azobis-(2-amidinopropan)hydrochlorid und 75 g Wasser wird Zulauf 2 hergestellt.

In einem 2 l-Glasgefäß, das mit Rührer, Heizung, Rückflußkühler und Dosiervorrichtungen ausgestattet ist, werden 290 g Wasser, 100 ml Zulauf 1 und 8 ml Zulauf 2 vorgelegt und unter Rühren auf 65° C erhitzt. Bei dieser Temperatur werden der restliche Zulauf 1 innerhalb von 5 Stunden und der restliche Zulauf 2 innerhalb von 7 Stunden zudosiert, Anschließend wird noch eine Stunde lange bei dieser Temperatur gerührt. Man erhält eine klare, viskose Polymerlösung. Der K-Wert des Polymeren beträgt 98,2.

Beispiel 7

Zulauf 1 besteht aus einer Mischung aus 855 g einer Monomerlösung gemäß Beispiel 4, 275 g Wasser und 40 g Isopropanol. Aus 4,6 g tert.-Butyl-2-ethylhexanoat und 157 g Isopropanol wird Zulauf 2 hergestellt. In einem 2 l-Glasgefäß, das mit Rührer, Heizung, Rückflußkühler und Dosiervorrichtungen ausgestattet ist, werden 40 g Wasser, 120 ml Zulauf 1 und 20 ml Zulauf 2 vorgelegt und bis zum schwachen Sieden unter Rückfluß erhitzt (ca. 83° C). Dann werden unter ständigem schwachen Sieden der restliche Zulauf 1 innerhalb von 6 Stunden, der restliche Zulauf 2 innerhalb von 8 Stunden zudosiert und anschließend wird noch eine Stunde lang am Sieden gehalten. Anschließend wird das Isopropanol durch Einleiten von Wasserdampf ausgetrieben. Man erhält eine klare, viskose Polymerlösung. Der K-Wert des Polymeren beträgt 57,8.

Beispiel 8

Zulauf 1 besteht aus einer Mischung aus 381 g einer Monomerlösung gemäß Beispiel 3, 75 g 2-Hydroxyethylacrylat und 180 g Wasser. Zulauf 2 wird aus 3 g 2,2'-Azobis(2-amidinopropan)hydrochlorid bereitet. In einem 2 l-Glasgefäß, das mit Rührer, Heizung, Rückflußkühler und Dosiervorrichtungen ausgestattet ist, werden 290 g Wasser, 100 ml Zulauf 1 und 8 ml Zulauf 2 vorgelegt und auf 65° C erwärmt. Bei dieser Temperatur werden der restliche Zulauf 1 innerhalb von 5 Stunden und der restliche Zulauf 2 innerhalb von 7 Stunden zudosiert. Danach wird noch eine Stunde lange bei dieser Temperatur gerührt. Man erhält eine trübe, viskose Polymerlösung. Der K-Wert des Polymeren beträgt 93.

Beispiel 9

Zulauf 1 besteht aus einer Mischung aus 203 g einer Monomerlösung gemäß Beispiel 3, 280 g Vinylpyrrolidon, 100 g Wasser und 1,2 g 2-Mercaptoethanol. Als Zulauf 2 dient eine Lösung von 8 g Wasserstoffperoxid (30%ig, in 75 g Wasser). In einem 2 l-Glasgefäß, das mit Rührer, Heizung, Rückflußkühler und Dosiervorrichtungen ausgestattet ist, werden 320 g Wasser, 50 ml Zulauf 1 und 10 ml Zulauf 2 vorgelegt und auf 65° C erhitzt. Bei dieser Temperatur werden der restliche Zulauf 1 innerhalb von 5 Stunden und der restliche Zulauf 2 innerhalb von 7 Stunden zudosiert. Es wird noch eine Stunde bei dieser Temperatur gerührt. Man erhält eine klare, viskose Polymerlösung. Der K-Wert des Polymeren beträgt 72,5.

Beispiel 10

Zulauf 1 besteht aus 330 g einer Monomerlösung gemäß Beispiel 4, 194 g Vinylpyrrolidon und 320 g Wasser, die mit konzentrierter Ammoniaklösung auf einen pH-Wert von 7,5 eingestellt wird. Der Zulauf 2 besteht aus einer Mischung von 2,8 g tert.-Butyl-2-ethylhexanoat in 110 g Isopropanol. In einem Glasgefäß, das mit Rührer, Heizung, Rückflußkühler und Dosiervorrichtungen ausgestattet ist, werden 142 g Isopropanol, 88 g Wasser, 140 ml Zulauf 1 und 10 ml Zulauf 2 vorgelegt und zum schwachen Sieden erhitzt (ca. 83° C). Dann werden bei dieser Temperatur der restliche Zulauf 1 innerhalb von 8 Stunden und der restliche Zulauf 2 innerhalb von 12 Stunden zudosiert. Danach wird noch 2 Stunden lang am Sieden gehalten. Anschließend wird bei Normaldruck das Isopropanol abdestilliert, bis eine Übergangstemperatur von 100° C erreicht wird. Man erhält eine klare, viskose Polymerlösung. Der K-Wert des Polymeren beträgt 85,0.

Beispiel 11

Zulauf 1 besteht aus einer Mischung aus 381 g einer Monomerlösung gemäß Beispiel 3, 75 g Hydroxypropylacrylat (technisches Isomerengemisch) und 180 g Wasser. Als Zulauf 2 dient eine Lösung von 3 g 2,2'-Azobis-(2-amidinopropan)hydrochlorid in 75 g Wasser. In einem Glasgefäß, das mit Rührer, Heizung, Rückflußkühler und Dosiervorrichtungen ausgestattet ist, werden 290 g Wasser, 100 ml Zulauf 1 und 8 ml Zulauf 2 vorgelegt und auf 65° C erwärmt. Bei dieser Temperatur werden der restliche Zulauf 1 innerhalb von 5 Stunden und der restliche Zulauf 2 innerhalb von 7 Stunden zudosiert. Es wird noch eine weitere Stunde bei 65° C gerührt. Man erhält eine schwach trübe, viskose Polymerlösung, die beim Verdünnen auf 20 % Feststoff klar wird. Der K-Wert des Polymeren beträgt 100,4.

Beispiel 12

Zulauf 1 besteht aus einer Mischung aus 203 g einer Monomerlösung gemäß Beispiel 3, 100 g Wasser, 280 g Vinylpyrrolidon und 1,2 g Mercaptoethanol. Als Zulauf 2 wird eine Lösung von 2,4 g Azobis(2,4-dimethylvaleronitril) in 75 g Ethanol verwendet. In einem 2 l-Glasgefäß, das mit Rührer, Heizung, Rückflußkühler und Dosiervorrichtungen ausgestattet ist, werden 320 g Wasser, 50 ml Zulauf 1 und 7 ml Zulauf 2 vorgelegt und auf 65° C erwärmt. Bei dieser Temperatur werden der restliche Zulauf 1 in 4 Stunden und der restliche Zulauf 2 in 6 Stunden zudosiert und anschließend wird noch eine Stunde gerührt. Das Ethanol wird durch Einleiten von Wasserdampf ausgetrieben. Man erhält eine klare, viskose Polymerlösung. Der K-Wert des Polymeren beträgt 78,3.

Beispiel 13

Zulauf 1 besteht aus einer Mischung aus 339 g einer Monomerlösung gemäß Beispiel 3, 200 g Vinylpyrrolidon, 96 g Wasser und 1 g 2-Mercaptoethanol, die mit konzentrierter Ammoniaklösung auf einen pH-Wert von 7,5 eingestellt wird. Als Zulauf 2 dient eine Lösung von 2,4 g 2,2'-Azobis-(2-amidinopropan)-hydrochlorid in 75 g Wasser. In einem 2 l-Glasgefäß, das mit Rührer, Heizung, Rückflußkühler und Dosiervorrichtungen ausgestattet ist, werden 196 g Wasser, 106 ml Zulauf 1 und 5 ml Zulauf 2 vorgelegt und auf 65° C erwärmt. Dann werden bei dieser Temperatur der restliche Zulauf 1 innerhalb von 5 Stunden und der restliche Zulauf 2 innerhalb von 7 Stunden zodosiert und noch eine Stunde nachgerührt. Man erhält eine klare, viskose Polymerlösung. Der K-Wert des Polymeren beträgt 78,0.

Beispiel 14

Zulauf 1 besteht aus einer Mischung aus 203 g einer Monomerlösung gemäß Beispiel 3, 100 g Wasser, 280 g Vinylpyrrolidon und 1,2 g Mercaptoethanol, die mit konzentrierter Ammoniaklösung auf pH 7,5 eingestellt wird. In einem 2 l-Glasgefäß, das mit Rührer, Heizung, Rückflußkühler und Dosiervorrichtungen versehen ist, werden 320 g Wasser, 50 ml Zulauf 1 und 0,2 g tert.-Butylperpivalat vorgelegt und auf 65° C erwärmt. Bei dieser Temperatur werden der restliche Zulauf 1 innerhalb von 4 Stunden zudosiert und parallel dazu alle 30 Minuten 0,2 g tert.-Butylperpivalat zugegeben, insgesamt 2,4 g innerhalb von 6 Stunden. Anschließend wird noch eine Stunde bei dieser Temperatur gerührt. Man erhält eine klare, viskose Polymerlösung. Der K-Wert des Polymeren beträgt 91.0.

Beispiel 15

Zulauf 1 besteht aus einer Mischung aus 203 einer Monomerlösung gemäß Beispiel 1, 125 g Wasser,

280 g Vinylpyrrolidon und 12 g Kaliumformiat. Zur Herstellung von Zulauf 2 werden 2 g 4,4'-Azobis-(cyanopentansäure) in 50 g Wasser suspendiert und mit ca. 5 ml 10%iger Natronlauge in Lösung gebracht. In einem Glasgefäß, das mit Rührer, Heizung, Rückflußkühler und Dosiervorrichtungen ausgestattet ist, werden 338 g Wasser, 50 ml Zulauf 1 und 5 ml Zulauf 2 vorgelegt und zum schwachen Sieden erhitzt (ca. 100 °C). Bei diesen Temperaturen werden der restliche Zulauf 1 innerhalb von 4 Stunden und der restliche Zulauf 2 innerhalb von 5 Stunden zudosiert und anschließend noch eine Stunde bei dieser Temperatur nachgerührt. Man erhält eine schwach trübe, viskose Polymerlösung. Der K-Wert des Polymeren beträgt 81,9.

Beispiel 16

Zulauf 1 besteht aus einer Mischung aus 381 g einer Monomerlösung gemäß Beispiel 3, 75 g 2-Hydroxyethylmethacrylat und 180 g Wasser. Als Zulauf 2 dient eine Lösung von 3 g 2,2'-Azobis(2-amidinopropan)hydrochlorid in 75 g Wasser. In einem Glasgefäß, das mit Rührer, Heizung, Rückflußkühler und Dosiervorrichtungen ausgestattet ist, werden 290 g Wasser, 100 ml Zulauf 1 und 8 ml Zulauf 2 vorgelegt und auf 65 °C erwärmt. Bei dieser Temperatur werden der restliche Zulauf 1 innerhalb von 5 Stunden und der restliche Zulauf 2 innerhalb von 7 Stunden zudosiert. Anschließend wird noch eine Stunde bei dieser Temperatur gerührt. Man erhält eine trübe, viskose Polymerlösung, die beim Verdünnen mit Wasser auf Festgehalte unter 5 Gew.-% klar wird. Der K-Wert des Polymeren beträgt 96,2.

## Ansprüche

1. Verfahren zur Herstellung von 3-Methyl-1-vinyl-imidazolium-chloriden der Formel I

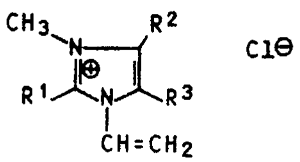

I,

in der $R^1$, $R^2$ und $R^3$ H oder Methyl bedeuten, dadurch gekennzeichnet, daß man eine 10 bis 70 Gew.%ige wäßrige Lösung eines N-Vinyl-imidazols der Formel II

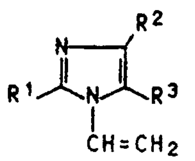

II,

in der $R^1$, $R^2$ und $R^3$ H oder Methyl bedeuten, bei Temperaturen von 40 bis 100 °C in einem geschlossenen System mit Methylchlorid in einer Menge von 90 bis 110 Mol%, bezogen auf das N-Vinylimidazol der Formel II, umsetzt.

2. Verfahren nach Anspruch 1, dadurch gekennzeichnet, daß man eine 25 bis 60 gew.%ige wäßrige Lösung eines N-Vinylimidazols der Formel II bei Temperaturen von 50 bis 90 °C mit Methylchlorid in einer Menge von 94 bis 100 Mol% umsetzt.

3. Verfahren nach Anspruch 1 oder 2, dadurch gekennzeichnet, daß die wäßrige Lösung des N-Vinylimidazols der Formel I, bezogen auf das Gewicht des Wassers, zusätzlich als Lösungsmittel 5 bis 30 Gew.-% eines einwertigen Alkohols mit 1 bis 3 C-Atomen enthält.

4. Verfahren zur Herstellung von Verbindungen der Formel I nach Anspruch 1, dadurch gekennzeichnet, daß man Verbindungen herstellt, in denen $R^1$ ein Wasserstoffatom oder Methyl und $R^2$ und $R^3$ jeweils ein Wasserstoffatom bedeuten.

5. Verwendung von 3-Methyl-1-vinylimidazolium-chloriden der Formel I in Form der nach den Ansprüchen 1 bis 3 erhaltenen Lösungen zur Herstellung von wasserlöslichen Homo- oder Copolymerisaten durch

radikalische Polymerisation in wäßriger oder wäßrig-alkoholischer Lösung bei Temperaturen von 40 bis 100 ° C.

## Claims

1. A process for the preparation of 3-methyl-1-vinylimidazolium chlorides of the formula I

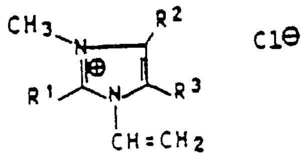

I

where $R^1$, $R^2$ and $R^3$ are each H or methyl, wherein a 10-70% strength by weight aqueous solution of an N-vinylimidazole of the formula II

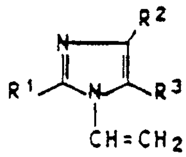

II

where $R^1$, $R^2$ and $R^3$ are each H or methyl, is reacted with methyl chloride in an amount of from 90 to 110 mol %, based on the N-vinylimidazole of the formula II, at from 40 to 100 ° C in a closed system.

2. A process as claimed in claim 1, wherein a 25-60% strength by weight aqueous solution of an N-vinylimidazole of the formula II is reacted with methyl chloride in an amount of from 94 to 100 mol % at from 50 to 90 ° C.

3. A process as claimed in claim 1 or 2, wherein the aqueous solution of the N-vinylimidazole of the formula I additionally contains from 5 to 35% by weight, based on the weight of the water, of a monohydric alcohol of 1 to 3 carbon atoms as a solvent.

4. A process for the preparation of a compound of the formula I as claimed in claim 1, wherein a compound is prepared in which $R^1$ is hydrogen or methyl and $R^2$ and $R^3$ are each hydrogen.

5. Use of a 3-methyl-1-vinylimidazolium chloride of the formula I, in the form of the solutions obtained as claimed in claim 1 or 2 or 3, for the preparation of water-soluble homo- or copolymers by free radical polymerization in aqueous or aqueous alcoholic solution at from 40 to 100 ° C.

## Revendications

1. Procédé de préparation de chlorures de 3-méthyl-1-vinyl-imidazolium de la formule I

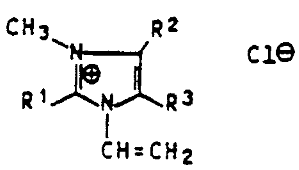

I

dans laquelle $R^1$, $R^2$ et $R^3$ représentent chacun un atome d'hydrogène ou le radical méthyle, caractérisé en ce que l'on fait réagir une solution aqueuse à 10-70% en poids d'un N-vinylimidazole de la formule II

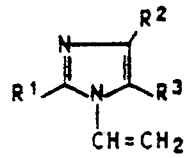

II

dans laquelle $R^1$, $R^2$ et $R^3$ représentent chacun un atome d'hydrogène ou le radical méthyle, à des températures de 40 à 100° C, dans un système fermé, sur le chlorure de méthyle, en une proportion de 90 à 100% molaires, par rapport au N-vinylimidazole de la formule II.

2. Procédé suivant la revendication 1, caractérisé en ce que l'on fait réagir une solution aqueuse à 60% d'un N-vinylimidazole de la formule II, à des températures de 50 à 90° C, sur le chlorure de méthyle, en une proportion de 94 à 100% molaires.

3. Procédé suivant la revendication 1 ou la revendication 2, caractérisé en ce que la solution aqueuse du N-vinylimidazole de la formule II contient, par rapport au poids de l'eau, complémentairement et à titre de solvant, 5 à 35% en poids d'un alcool monohydroxylé qui comporte de 1 à 3 atomes de carbone.

4. Procédé de préparation de composés de la formule I selon la revendication 1, caractérisé en ce que l'on prépare des composés dans lesquels $R^1$ représente un atome d'hydrogène ou le radical méthyle et $R^2$ et $R^3$ représentent chacun un atome d'hydrogène.

5. Utilisation de chlorures de 3-méthyl-1-vinylimidazolium de la formule I sous forme des solutions obtenues selon les revendications 1 à 3, en vue de la préparation d'homopolymères ou de copolymères solubles dans l'eau par la polymérisation radicalaire en solution aqueuse ou en solution aqueuse-alcoolique, à des températures de 40 à 100° C.